# EUROPÄISCHE PATENTSCHRIFT

(11) **EP 0 581 222 B1**
(45) Veröffentlichungstag und Bekanntmachung des Hinweises auf die Patenterteilung: **31.01.1996**
(21) Anmeldenummer: 93111888.9
(22) Anmeldetag: 24.07.1993
(51) Int. Cl.: C07C 45/78, C07C 47/22, C07C 45/35

(54) **Verfahren zur Abtrennung von Methacrolein aus einem gasförmigen Gemisch**
Process for separating methacrolein from a gaseous mixture
Procédé pour la séparation de méthacroleine d'un mélange gazeux

(30) Priorität: 31.07.1992 DE 4225321
(43) Veröffentlichungstag der Anmeldung: 02.02.1994
(73) Patentinhaber: BASF Aktiengesellschaft, D-67063 Ludwigshafen (DE)
(72) Erfinder: Vogel, Herbert, Dr., D-6700 Ludwigshafen (DE); Exner, Herbert, Dr., D-6701 Waldsee (DE); Bohner, Gerd, D-76669 Bad Schoenborn (DE)

(56) Entgegenhaltungen:
- GB-A- 2 004 886
- CHEMICAL ABSTRACTS, vol. 112, no. 12, 19. März 1990, Columbus, Ohio, US; abstract no. 99474y, KURAGANO, MORIMASA ET AL. 'Absorption of methacrolein by aqueous methacrylic acid.' Seite 15 ;Spalte 1 ;
- CHEMICAL ABSTRACTS, vol. 112, no. 12, 19. März 1990, Columbus, Ohio, US; abstract no. 99474y, KURAGANO, MORIMASA ET AL. 'Absorption of methacrolein by aqueous methacrylic acid.' Seite 15 ;Spalte 1 ; & JP-A-01 242 547

## Beschreibung

Die vorliegende Erfindung betrifft ein Verfahren zur Abtrennung von Methacrolein aus einem gasförmigen Gemisch durch Absorption mittels einer Methacrylsäure enthaltenden wäßrigen Lösung.

Methacrolein kann unter anderem durch katalytische Gasphasenoxidation von C₄-Verbindungen wie Butan, iso-Buten, tert.-Butanol oder iso-Butyraldehyd erzeugt werden (vgl. z.B. DE-A 28 42 092). Im Rahmen dieser Herstellverfahren wird jedoch nicht reines Methacrolein sondern ein gasförmiges Gemisch erhalten, aus welchem das Methacrolein zur Herstellung von Folgeprodukten in der Regel abgetrennt werden muß. Aber auch dann, wenn man das bei der katalytischen Gasphasenoxidation von Methacrolein anfallende Gasgemisch unmittelbar zur Herstellung der Folgeprodukte einsetzen kann, stellt sich häufig die Frage der Abtrennung von nicht umgesetztem Methacrolein aus dem das Folgeprodukt enthaltenden Gasgemisch. Dies gilt insbesondere im Fall der Umsetzung des Methacrolein zu Methacrylsäure, welche, ebenso wie die Methacroleinherstellung selbst, in vorteilhafter Weise nach der Methode der katalytischen Gasphasenoxidation durchgeführt wird, bei der jedoch unter den Reaktionsbedingungen, unter denen die Selektivität der Methacrylsäurebildung hoch ist, mit den meisten derzeit verfügbaren Katalysatoren ein relativ niedriger Methacroleinumsatz einhergeht, weshalb eine Abtrennung des nicht umgesetzten Methacroleins vom gasförmigen Reaktionsgemisch und dessen Rückführung in den Oxidationsreaktor insbesondere hinsichtlich der Wirtschaftlichkeit des Gesamtverfahrens einen wesentlichen Faktor darstellt.

Aus der DE-A 28 42 092 ist bekannt, Methacrolein aus derartigen gasförmigen Gemischen in Anwendung eines flüssigen Absorbens zunächst zu absorbieren und aus der dabei anfallenden, das Methacrolein enthaltenden, Lösung das Methacrolein destillativ oder durch Abstreifen Zu gewinnen. Als Absorbens wird die Verwendung einer wäßrigen Methacrylsäure enthaltenden Lösung empfohlen, deren Methacrylsäuregehalt wenigstens 5 Gew.-% beträgt.

Der Vorteil der Verwendung einer wäßrigen Methacrylsäurelösung als Absorbens besteht u.a. darin, daß Methacrylsäure mit Methacrolein artverwandt ist und in der Regel bei Folgereaktionen des Methacroleins, das in der Regel nach der Abtrennung mit Spuren des verwendeten Absorbens behaftet ist, nicht wesentlich stört.

Aus der US-A 4,618,709 ist ferner bekannt, daß der Absorptionskoeffizient einer Methacrylsäure enthaltenden wäßrigen Lösung für Methacrolein mit steigendem Gehalt an Methacrylsäure zunimmt. Methacrylsäure gehört jedoch zur Klasse der α,β-monoethylenisch ungesättigten Verbindungen, welche in der Regel eine ausgeprägte Polymerisationsneigung aufweisen, die, in Lösung befindlich, üblicherweise mit zunehmendem Gewichtsanteil zunimmt. Dem kann zwar durch Zusatz einer erhöhten Menge an Polymerisationsinhibitoren begegnet werden, jedoch ist die Mitverwendung erhöhter Anteile an Fremdsubstanz generell unerwünscht. Man findet daher im Stand der Technik zur Absorption von Methacrolein mittels Methacrylsäure enthaltenden wäßrigen Lösungen ausschließlich die Verwendung solcher Lösungen, deren Gehalt an Methacrylsäure ≦ 45 Gew.-% beträgt (DE-A 28 42 092 und US-A 4,618,709).

Nachteilig an diesen Verfahrensweisen im Stand der Technik ist jedoch der nicht befriedigende Absorptionskoeffizient für Methacrolein, dem im Stand der Technik dadurch zu begegnen versucht wird, daß
a) die Absorption bei tiefen Temperaturen und/oder unter Anwendung erhöhter Mengen Absorptionsmittel durchgeführt (DE-A 28 42 092) oder
b) der Zusatz von Essigsäure als den Absorptionskoeffizienten für Methacrolein erhöhendes Mittel empfohlen wird (US-A 4,618,709).

Von Nachteil an diesen Hilfsmaßnahmen ist jedoch, daß bei niederen Temperaturen in Methacrolein enthaltenden verdünnten wäßrigen Methacrylsäurelösungen eine Phasentrennung auftritt, von denen eine der beiden Phasen Methacrolein in erhöhter Konzentration enthält. Letzteres ist insofern von Nachteil, als mit einem zunehmenden Methacroleingehalt eine erhöhte Polymerisationsneigung desselben einhergeht.

Die notwendige Mitverwendung von Essigsäure ist insofern von Nachteil, als dabei eine weitere Fremdsubstanz eingebracht wird.

Die Aufgabe der vorliegenden Erfindung bestand daher darin, ein Verfahren zur Absorption von Methacrolein aus einem gasförmigen Gemisch zur Verfügung zu stellen, das die genannten Nachteile nicht aufweist.

Demgemäß wurde ein Verfahren zur Abtrennung von Methacrolein aus einem gasförmigen Gemisch durch Absorption mittels einer Methacrylsäure enthaltenden wäßrigen Lösung gefunden, das dadurch gekennzeichnet ist, daß die wäßrige Lösung, bezogen auf ihr Gesamtgewicht, 60 bis 90 Gew.-% Methacrylsäure enthält.

Grundlage des erfindungsgemäßen Verfahrens bildet die Feststellung, daß die Polymerisationsneigung einer wäßrigen Methacrylsäurelösung bei einem Methacrylsäuregehalt von ≧ 60 Gew.-% in überraschender Weise wesentlich geringer ist, als bei einem Methacrylsäuregehalt im Bereich um 40 Gew.-%.

Eine weitere bemerkenswerte Eigenschaft wäßriger Methacrylsäurelösungen mit einem Methacrylsäuregehalt von 60 bis 90 Gew.-%, im Vergleich zu entsprechenden Lösungen mit einem geringeren Methacrylsäuregehalt, besteht darin, daß ihre Neigung zur Phasentrennung bei Absorption von Methacrolein auch bei tiefen Temperaturen sehr viel weniger ausgeprägt ist, bzw. daß eine solche Phasentrennung überhaupt nicht eintritt. Letzteres trifft beispielsweise auf eine 60 gew.-%ige wäßrige Methacrylsäurelösung bei 20°C und 1 atm zu, nicht jedoch auf eine ≦ 50 gew.-%ige wäßrige Methacrylsäurelösung unter gleichen Bedingungen.

Das erfindungsgemäße Verfahren ist somit im Vergleich zu den Verfahren im Stand der Technik insbesondere insofern von Vorteil, als das verwendete Absorbens für Methacrolein bei gegebener Absorptionstemperatur einen erhöhten Absorptionskoeffizienten aufweist und gleichzeitig die Möglichkeit eröffnet, ohne Phasentrennung bei tieferen Temperaturen zu absorbieren, was eine weitere Steigerung des Absorptionskoeffizienten bedingt.

In anwendungstechnisch vorteilhafter Weise wird man das erfindungsgemäße Absorptionsverfahren in der Regel so durchführen, daß in der verwendeten Absorptionsvorrichtung die Temperatur 5 bis 50, bevorzugt 20 bis 40°C, und ganz besonders bevorzugt 30 bis 35°C` beträgt, was die Mitverwendung aufwendiger Kühlvorrichtungen und den damit einhergehenden Energieaufwand reduziert. Die Abtrennung des Methacroleins aus der anfallenden Absorptionslösung kann beispielsweise destillativ und/oder durch Abstreifen (strippen) erfolgen.

Hinsichtlich einer destillativen Abtrennung des Methacroleins erweist es sich als besonders vorteilhaft, daß der Siedepunkt wäßriger Methacrylsäurelösungen bei Normaldruck über einen weiten Konzentrationsbereich vom Methacrylsäuregehalt nahezu unabhängig ist (vgl. Zh. Prikl. Khim. 47, 2130 (1974)) und erst oberhalb von 85 Gew.-% deutlich ansteigt. Im Bereich von 30 bis 85 Gew.-% liegt der Siedpunkt bei 760 Torr im wesentlichen bei 100°C. Vorzugsweise erfolgt das erfindungsgemäße Verfahren daher in Anwendung einer wäßrigen Methacrylsäure enthaltenden Lösung, deren Methacrylsäuregehalt 60 bis 80, besonders bevorzugt 60 bis 70 Gew.-% beträgt. Die nach der Abtrennung des Methacroleins verbleibende wäßrige Methacrylsäurelösung kann zur weiteren Absorption wiederverwendet werden.

Die Anwesenheit zusätzlicher Carbonsäuren im Absorbens, z.B. Essigsäure, erweist sich in der Regel nicht als störend. So bewirkt die Anwesenheit geringer Mengen Essigsäure beispielsweise eine zusätzliche Reduktion der Neigung zur Phasentrennung. Der Gewichtsanteil an Essigsäure, bezogen auf die zur Absorption des Methacroleins aufgegebene wäßrige Gesamtlösung, sollte jedoch 20, vorzugsweise 5 Gew.-%, nicht überschreiten.

Das Auswaschen des Methacrolein enthaltenden Gasgemisches erfolgt ansonsten in dem Durchschnittsfachmann an sich bekannter Weise. Beispielsweise kann das Auswaschen in einer oder mehreren hintereinander geschalteten Absorptionsvorrichtungen durchgeführt werden. Im allgemeinen ist die Anwendung einer Vorrichtung ausreichend. Vorzugsweise wird die Absorption im Gegenstrom geführt, wenngleich auch ein Kontakt in Parallelströmung angewendet werden kann, jedoch weniger zweckmäßig ist. Die Zone, in der der Kontakt zwischen dem gasförmigen Gemisch und dem Absorbens stattfindet, kann von herkömmlicher, für einen Kontakt zwischen einem Gas und einer Flüssigkeit geeigneter Ausgestaltung sein. Die Art der dabei verwendeten Absorptionsvorrichtung unterliegt keiner Beschränkung. Beispielsweise können beliebige herkömmliche Absorptionstürme wie Füllkörper-, Siebboden-, Glockenboden- oder Sprühtürme eingesetzt werden. Damit das zu waschende Gemisch leicht durch die Auswaschzonen strömt, befindet es sich in zweckmäßiger Weise auf erhöhtem Druck. Die Obergrenze des Druckes ergibt sich in erster Linie aus wirtschaftlichen Erwägungen. Normalerweise ist ein Überdruck von > 0 bis 10⁵ Pa zweckmäßig. Vorzugsweise liegt der Überdruck bei 2·10⁴ Pa. Die für die Methacroleinabsorption zu verwendende Menge an Methacrylsäure enthaltender wäßriger Lösung richtet sich u.a. nach dem Methacroleingehalt des zu waschenden Gasgemisches. Sie kann vom Fachmann in wenigen Versuchen in einfacher Weise ermittelt werden.

In besonders vorteilhafter Weise eignet sich das erfindungsgemäße Verfahren zur Abtrennung von Methacrolein aus Gasgemischen, die bei der katalytischen Gasphasenoxidation von C₄-Verbindungen zu Methacrolein erhalten werden. Ferner ist es besonders vorteilhaft zur Abtrennung von nicht umgesetztem Methacrolein aus Gasgemischen, die bei der katalytischen Gasphasenoxidation von Methacrolein, oder bei über Methacrolein als intermediäre Verbindung führende katalytische Gasphasenoxidation von C₄-Verbindungen zu Methacrylsäure erhalten werden.

Bei Verfahren dieser Art werden Verbindungen wie Butan, iso-Buten, tert.-Butanol oder iso-Butyraldehyd tpyischer Weise in einem Festbettrohrbündelreaktor in Gegenwart eines Multimetalloxidkatalysators bei Temperaturen, die im allgemeinen zwischen 250 und 550°C liegen, mit Sauerstoff zu Methacrolein oder in zwei zeitlich aufeinanderfolgenden Stufen (die räumlich getrennt oder vereint realisiert sein können) zu Methacrylsäure oxidiert. Üblicherweise werden die reaktiven Gaskomponenten bei diesen exothermen Umsetzungen aus Gründen einer besseren Reaktionskontrolle und Wärmeabfuhr in Gegenwart großer Mengen Verdünnungsgase umgesetzt. Als solche Verdünnungsgase kommen inerte Gase wie Stickstoff und Kohlendioxid, aber auch Wasserdampf in Betracht.

Das gasförmige Reaktionsprodukt enthält daher außer Methacrolein und gegebenenfalls Methacrylsäure vor allem große Mengen Verdünnungsgase, nicht umgesetzte Ausgangsverbindungen sowie geringe Mengen organischer Nebenprodukte wie Aldehyde, Ketone oder aliphatische Carbonsäuren (z.B. Essigsäure). In der Regel liegt der Methacroleinanteil unter 5 Vol.-%. Um den Methacroleinanteil bereits vor Anwendung des erfindungsgemäßen Verfahrens anzureichern, wird das Reaktionsgemisch vorzugsweise einem oder mehreren Kondensationsschritten unterworfen. Dabei können die Bestandteile abgetrennt werden, die einen höheren Siedepunkt als Methacrolein aufweisen. Danach wird das dabei resultierende Gasgemisch in einer geeigneten Absorptionsvorrichtung dem erfindungsgemäßen Verfahren unterworfen.

Aus der dabei resultierenden Absorptionslösung kann anschließend das Methacrolein z.B. durch Abstreifen mit einem Inertgas abgetrennt und gemeinsam mit dem Inertgas unmittelbar der katalytischen Gasphasenoxidation wieder zugeführt oder durch fraktionierte Destillation rein gewonnen und als solches verschiedensten Folgereaktionen zugeführt werden. Selbstverständlich erfolgen auch die erfindungsgemäßen Verfahrensschritte in Gegenwart von wirksamen Mengen an Polymerisationsinhibitoren wie Hydrochinon, Hydrochinonmonomethylether oder Phenothiazin. Die verwendeten Einsatzmengen bewegen sich üblicherweise, bezogen auf die α,β-monoethylenisch ungesättigten Verbindungen, im Bereich von 50 bis 1000 ppm.

### Beispiele

### Beispiel 1

Untersuchung der Polymerisationsneigung verschiedener, einen unterschiedlichen Gehalt an Methacrylsäure aufweisenden, Methacrylsäurelösungen.

Die wäßrigen Methacrylsäurelösungen (hergestellt durch Mischen von entsprechenden Mengen Wasser und einer Methacrylsäure, deren Gehalt an organischen Verunreinigungen ≦ 0,1 Gew.-% betrug) wurden unter Rückfluß am Sieden gehalten und in Abhängigkeit von der Zeitdauer ihr Viskositätsverhalten visuell beurteilt. Sie enthielten in allen Fällen, bezogen auf die enthaltene Menge Methacrylsäure, 170 ppm Hydrochinonmonomethylether als Polymerisationsinhibitor.

Ergebnisse:
a) 75 gew.-%ige wäßrige Methacrylsäurelösung
   Siedetemperatur: 98°C
   Nach sechs Tagen war noch keine wesentliche Änderung der Viskosität feststellbar.
b) 60 gew.-%ige wäßrige Methacrylsäurelösung
   Siedetemperatur: 98°C
   Nach vier Tagen beginnende Viskositätsänderung feststellbar.
c) 40 gew.-%ige wäßrige Methacrylsäurelösung
   Siedetemperatur: 98°C
   Bereits nach zwei Tagen war ein deutlicher Viskositätsanstieg zu beobachten.

### Beispiel 2

Erfindungsgemäße Absorption von Methacrolein aus einem Gasgemisch (verwendeter Inhibitor: Hydrochinon).

Tert.-Butanol wurde mit in Luft enthaltenem Sauerstoff in Gegenwart von Wasserdampf als weiterem Verdünnungsgas in einem Festbettreaktor durch katalytische Gasphasenoxidation zu Methacrolein aufoxidiert. Das 250°C heiße Produktgemisch enthielt 2,5 Vol.-% Methacrolein, 0,4 Vol.-% Methacrylsäure und im übrigen andere Nebenprodukte, wie geringe Mengen an Essigsäure sowie die Verdünnungsgase.

In mehreren Kondensationsschritten, deren letzter bei 30°C betrieben wurde, wurden höher siedene Komponenten auskondensiert.

Das Restgasgemisch, das einen Methacroleingehalt von 2,9 Vol.-% aufwies, wurde mit einer Geschwindigkeit von 2 m³/h im Gegenstrom einer Absorptionskolonne zugeführt, die bei ca. 30°C mit einer 60 gew.-%igen wäßrigen Methacrylsäurelösung als Absorbens betrieben wurde.

Die resultierende Absorptionslösung enthielt 5,1 Gew.-% Methacrolein. Sie wurde zur destillativen Trennung einer Füllkörperkolonne zugeführt.

Das Kopfprodukt enthielt Methacrolein, geringe Mengen Leichtsieder wie Acetaldehyd, Aceton und Acrolein sowie Wasser. Nach Abkühlen trennte sich das Kopfprodukt in zwei Phasen, von denen die überschüssige Phase aus ca. 92 gew.-%igem Methacrolein bestand.

## Patentansprüche

1. Verfahren zur Abtrennung von Methacrolein aus einem gasförmigen Gemisch durch Absorption mittels einer Methacrylsäure enthaltenden wäßrigen Lösung, dadurch gekennzeichnet, daß der Gehalt der wäßrigen Lösung an Methacrylsäure 60 bis 90 Gew.-% beträgt.

2. Verfahren nach Anspruch 1, dadurch gekennzeichnet, daß die Absorptionstemperatur 5 bis 50°C beträgt.

3. Verfahren nach den Ansprüchen 1 oder 2, dadurch gekennzeichnet, daß das Methacrolein enthaltende Gemisch ein solches ist, das durch katalytische Gasphasenoxidation einer vier Kohlenstoffatome aufweisenden organischen Verbindung zu hauptsächlich Methacrolein und/oder Methacrylsäure erhalten worden ist.

## Claims

1. Process for removing methacrolein from a gaseous mixture by absorption by means of an aqueous solution containing methacrylic acid, wherein the content of methacrylic acid in the aqueous solution is from 60 to 90% by weight.

2. A process as claimed in claim 1, wherein the absorption is carried out at from 5 to 50°C.

3. A process as claimed in claim 1 or 2, wherein the methacrolein-containing mixture has been obtained from the catalytic gas-phase oxidation of a four-carbon organic compound to, principally, methacrolein and/or methacrylic acid.

## Revendications

1. Procédé de séparation de la méthacroléine d'un mélange gazeux par absorption à l'aide d'une solution aqueuse contenant de l'acide méthacrylique, caractérisé en ce que la teneur de la solution aqueuse en acide méthacrylique varie de 60 à 90% en poids.

2. Procédé suivant la revendication 1, caractérisé en ce que la température d'absorption varie de 5 à 50°C.

3. Procédé suivant l'une quelconque des revendications 1 et 2, caractérisé en ce que le mélange contenant de la méthacroléine en est un que l'on a obtenu par l'oxydation catalytique en phase gazeuse d'un composé organique présentant 4 atomes de carbone principalement en méthacroléine et/ou acide méthacrylique.
